(19) Europäisches Patentamt
European Patent Office
Office européen des brevets

(11) Veröffentlichungsnummer: **0 249 999**
**A2**

# (12) EUROPÄISCHE PATENTANMELDUNG

(21) Anmeldenummer: **87108841.5**

(22) Anmeldetag: **20.06.87**

(51) Int. Cl.⁴: **C07D 249/08**, A01N 43/653

(30) Priorität: **20.06.86 DE 3620657**

(43) Veröffentlichungstag der Anmeldung:
**23.12.87 Patentblatt 87/52**

(84) Benannte Vertragsstaaten:
**AT BE CH DE ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Wenderoth, Bernd, Dr.**
**Schwalbenstrasse 26**
**D-6840 Lampertheim(DE)**
Erfinder: **Rentzea, Costin, Dr.**
**Richard-Kuhn-Strasse 1-3**
**D-6900 Heidelberg(DE)**
Erfinder: **Ammermann, Eberhard, Dr.**
**Sachsenstrasse 3**
**D-6700 Ludwigshafen(DE)**
Erfinder: **Pommer, Ernst-Heinrich, Dr.**
**Berliner Platz 7**
**D-6703 Limburgerhof(DE)**

## (54) Triazolylethylether und diese enthaltende Fungizide.

(57) Verbindungen der Formel

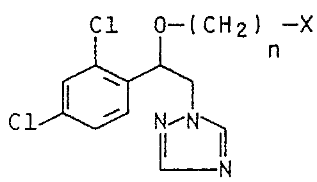

in der
n eine ganze Zahl von 2 bis 6 bedeutet und
X Halogen, Cyano, Thiocyano, Alkoxy, gegebenenfalls alkylsubstituiertes Cycloalkoxy, ein substituiertes Amin, einen heterocyclischen Rest oder einen Aroyloxyrest der Formel

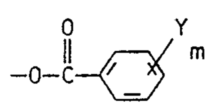

bedeutet, wobei Y für Halogen, Trifluormethyl, Nitro, Alkyl oder Alkoxy steht und m für eine ganze Zahl von 0 bis 5 steht und deren Salze und Komplexverbindungen und diese Verbindungen enthaltende Fungizide.

EP 0 249 999 A2

# Triazolylethylether und diese enthaltende Fungizide

Die vorliegende Erfindung betrifft neue Triazolylethylether, ihre Herstellung, ihre Verwendung als Fungizide und Fungizide.

Es ist bekannt, daß Azolderivate wie z.B. das 1-[2-(2,4-Dichlorphenyl)-2-(propenyloxy)-ethyl]-1H-imidazol (GB 1 218 590) fungizide Wirksamkeit besitzen. Die Wirkung dieser Verbindungen ist jedoch nicht befriedigend.

Es wurde nun gefunden, daß Triazolverbindungen der Formel I

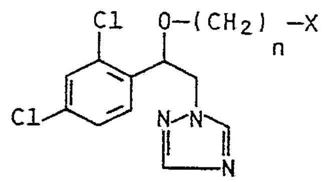

(I)

in der

n eine ganze Zahl von 2 bis 6 bedeutet und

X Halogen, Cyano, Thiocyano, gegebenenfalls $C_1$-$C_4$-alkylsubstituiertes $C_4$-$C_6$-Alkoxy, gegebenenfalls $C_1$-$C_4$-alkylsubstituiertes $C_5$-$C_8$-Cycloalkoxy, ein Amin der Formel

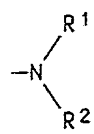

wobei $R^1$ und $R^2$ gleich oder verschieden sind und Benzyl, $C_2$-$C_4$-Alkenyl oder $C_1$-$C_4$-Alkyl bedeuten, die gegebenenfalls zu einem gegebenenfalls $C_1$-$C_4$-alkyl-oder $C_6$-$C_{10}$-arylsubstituierten Ring geschlossen sein können, der zusätzlich ein Stickstoff-oder Sauerstoffatom im Ring enthalten kann oder $-NR^1R^2$ einen Imidazolyl-oder Triazolylrest bedeutet,

oder einen Aroyloxyrest der Formel

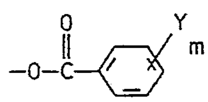

bedeutet, wobei Y für Halogen, Trifluormethyl, Nitro, $C_1$-$C_4$-Alkyl oder $C_1$-$C_4$-Alkoxy steht und m für eine ganze Zahl von 0 bis 5 steht, sowie deren für Pflanzen verträgliche Säureadditionssalze und Metallkomplexverbindungen neben einer sehr hohen fungitoxischen Wirkung auch eine ausgezeichnete Pflanzenverträglichkeit zeigen.

X bedeutet beispielsweise Halogen (Fluor, Chlor, Brom), $C_4$-$C_6$-Alkoxy (Butoxy, Pentoxy, Hexoxy), ein-oder mehrfach methyl-und/oder ethylsubstituiertes $C_4$-$C_6$-Alkoxy (Butoxy, Pentoxy, Hexoxy), $C_5$-$C_8$-Cycloalkoxy (Cyclopentoxy, Cyclohexoxy, Cyclooctoxy), Imidazol, Triazol (1,2,4,-Triazol-1-yl), Diethylamin, Di-n-butylamin, Diallylamin, Benzyl-methylamin oder

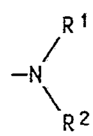

bedeutet, wobei $R^1$ und $R^2$ zusammen mit dem Stickstoff einen 5 bis 7-gliedrigen heterocyclischen Ring bedeuten, der gegebenenfalls durch $C_1$-$C_4$-Alkyl (Methyl, Ethyl, Propyl, Butyl) oder $C_6$-$C_{10}$-Aryl (Phenyl, Naphthyl) substituiert ist und der gegebenenfalls zusätzlich zu dem Stickstoffatom ein weiteres Stickstoffatom oder ein Sauerstoffatom im Ring trägt, z.B. Pyrrolidin, 2-Methylpyrrolidin, Piperidin, 2-Methylpiperidin,

4-Methylpiperidin, 4-t-Butylpiperidin, 4-Phenylpiperidin, Morpholin, 2,6-Dimethylmorpholin, Hexamethylenimin, N-Methylpiperazin, oder enen Benzoyloxyrest bedeutet, wobei der Benzylrest gegebenenfalls substituiert ist durch $C_1$-$C_4$-Alkyl (Methyl, Ethyl, Propyl, Butyl) oder durch $C_1$-$C_4$-Alkoxy (Methoxy, Ethoxy, Propoxy, Butoxy) z.B. Benzoyloxy, 3-Methylbenzoyloxy, 3-Ethylbenzoyloxy, 4-Butylbenzoyloxy, 3-Trifluormethylbenzoyloxy, 4-Methoxybenzoyloxy, 4-Ethoxybenzoyloxy, 3-Flurobenzoyloxy, 4-Fluorbenzoyloxy, 4-Chlorbenzoyloxy, 2,4-Dichlorbenzoyloxy oder 3-Nitrobenzoyloxy.

Die Verbindungen der Formel I können in üblicher Weise in für Pflanzen verträgliche Salze oder Metallkomplexe überführt werden. Zur Salzbildung eignen sich Mineralsäuren, wie Salzsäure, Bromwasserstoffsäure, Schwefelsäure, Salpetersäure, Phosphorsäure und organische Säuren, wie Essigsäure und Dodecylbenzolsulfonsäure. Die fungizide Wirksamkeit der Salze beruht auf dem Kation, so daß das Anion beliebig aus der Vielzahl der pflanzenphysiologisch verträglichen Anionen ausgewählt werden kann.

Metallkomplexe können durch Anlagerung an die Kationen von Metallsalzen gebildet werden. Hierfür eignen sich insbesondere Kupfer-, Zink-, Eisen-, Mangan-und Nickelsalze, z.B. Kupfer(II)-chlorid, Kupfer(II)-sulfat, Kupfer(III)-nitrat, Zink(II)-chlorid, Eisen(III)-chlorid, Mangan(II)-chlorid, Nickel(II)-bromid.

Die Verbindungen der Formel I lassen sich z.B. herstellen, indem man z.B. einem der unten aufgeführten Schemata folgt:

Schema A

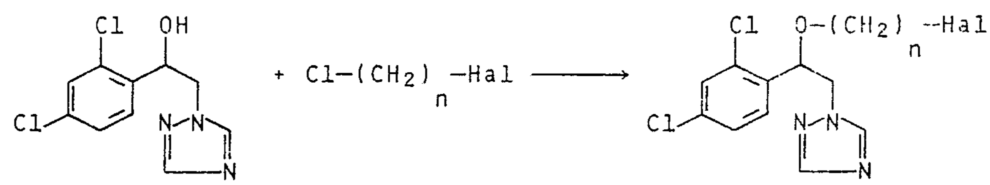

(II) (III), Hal = F, Cl, Br, CN, SCN

Schema B

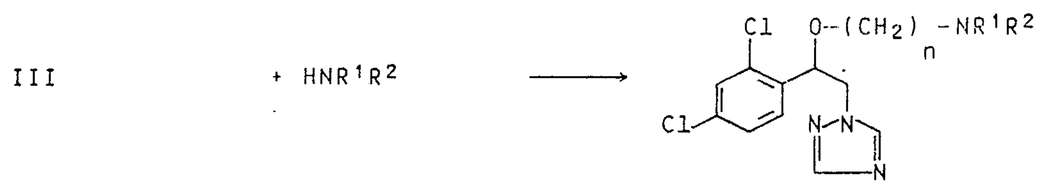


Schema C

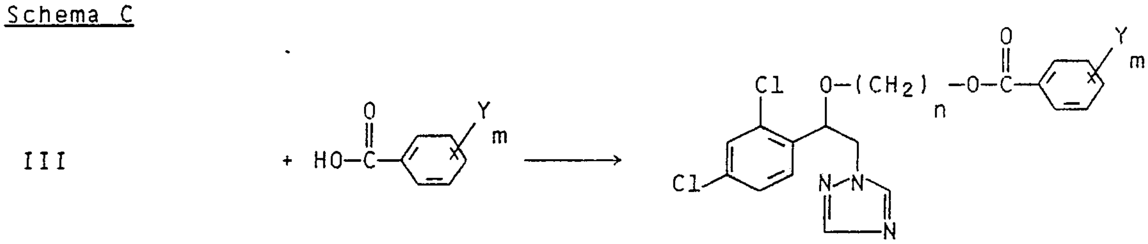


Schema D

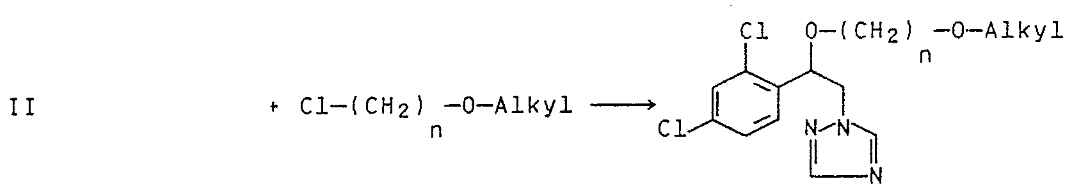

Das bekannte Ausgangsmaterial der Formel II läßt sich nach bekannten Methoden leicht herstellen (DE-OS 25 56 319).

Die Reaktionen können gegebenenfalls in Gegenwart eines Verdünnungsmittels und/oder eines Katalysators durchgeführt werden. Als Verdünnungsmittel können z.B. Chloroform, Methylenchlorid, Toluol, Xylole, Diethylether, Tetrahydrofuran, Dioxan, Dimethylsulfoxid, Dimethylformamid, Methanol, Ethanol oder tert.-Butanol verwendet werden. Zweckmäßige Katalysatoren sind z.B. Amine, wie z.B. Triethylamin oder Pyrrolidin, Metallhydroxide wie Natriumhydroxid oder Kaliumhydroxid, Metallhalogenide wie Kaliumjodid und Ammoniumverbindungen wie Tetrabutylammoniumhydrogensulfat.

Die Herstellung der neuen Verbindungen der Formel I wird durch folgende Beispiele erläutert:

Beispiel 1 (Verb.-Nr. 2)

60 g (0,23 mol) 1-(2,4-Dichlorphenyl)-2-(1,2,4-triazol-1-yl)-ethan-1-ol, 500 ml 1,5-Dichlorpentan, 11,5 g Tetrabutylammoniumhydrogensulfat als Phasentransfer-Katalysator und 140 ml konzentrierte Natronlauge als Gegenphase werden 16 h bei Raumtemperatur kräftig gerührt. Anschließend wird auf $H_2O$ gegossen und mehrmals mit Dichlormethan extrahiert. Die vereinigten organischen Phasen werden mit $H_2O$ neutral gewaschen und über $Na_2SO_4$ getrocknet. Sie werden im Vakuum eingeengt, um überschüssiges 1,5-Dichlorpentan vollständig zu entfernen. Man erhält 68 g (82 %) 1,(5-Chlorpentoxy)-1-(2,4-dichlorphenyl)-2-(1,2,4-triazol-1-yl)-ethan als Öl.

$^1$H-NMR ($CDCl_3$) : $\delta$ = 1.4 (m, 2H), 1.5 (m, 2H), 1.7 (m, 2H),
3.2 (m, 1H), 3.4 (m, 1H), 3.5 (t, 2H),
4.2 (m, 1H), 4.45 (m, 1H), 5.05 (m, 1H)
7.2-7.5 (m, 3H), 7.9 (s, 1H), 8.15 (s, 1H).

Beispiel 2 (Verb.-Nr. 22)

10 g (28 mmol) 1-(5-Chlorpentoxy)-1-(2,4-dichlorphenyl)-2-(1,2,4-triazol-1-yl)-ethan und eine Spatelspitze Kaliumjodid werden in 70 ml Pyrrolidin unter Rückfluß erhitzt. Nach 16 h wird im Vakuum eingeengt und der Rückstand mit Diethylether gelöst und mit $H_2O$ neutral gewaschen und die Lösung über $Na_2SO_4$ getrocknet. Nach dem Abdampfen des Lösungsmittels werden 9 g (82 %) 1-(2,4-Dichlorphenyl)-1-[5-(pyrrolidin-1-yl)-pentoxy]-2-(1,2,4-triazol-1-yl)-ethan erhalten.

$^1$H-NMR ($CDCl_3$): $\delta$ = 1.2 (m, 2H), 1.5 (m, 4H), 1.75 (s, 4H),
2.35 (t, 2H), 2.45 (s, 4H), 3.2 (m, 1H),
3.35 (m, 1H), 4.2 (m, 1H), 4.45 (m, 1H),
5.0 (m, 1H), 7.2-7.5 (m, 3H),
7.9 (s, 1H), 8.15 (s, 1H).

Beispiel 3 (Verb.-Nr. 58)

Zu 3,2 g (57 mmol) gepulvertem Kaliumhydroxid suspendiert in 100 ml absolutem Ethanol werden unter Rühren 9 g (57 mmol) 4-Chlorbenzoesäure (gelöst in 50 ml abs. Ethanol) bei Raumtemperatur zugetropft. Nach zweistündigem Rühren wird das Lösungsmittel entfernt und noch eine Stunde im Ölpumpen-Vakuum getrocknet. Anschließend versetzt man mit 100 ml absolutem Dimethylformamid (DMF) und einer Spatelspitze Kaliumjodid und tropft unter Rühren eine Lösung von 12 g (34 mmol) 1(4-Chlorbutoxy)-1-(2,4-dichlorphenyl)-2-(1,2,4-triazol-1-yl)-ethan in 50 ml absolutem DMF zu. Dann wird 8 h bei 100°C gerührt, das Lösungsmittel im Vakuum entfernt und der Rückstand in Ether aufgenommen.

Es wird mit $H_2O$ gewaschen, über $Na_2SO_4$ getrocknet und der Ether entfernt. Man erhält 12 g (75 %) 1-[4-(4-Chlorbenzoyloxy)-butoxy]-1-(2,4-dichlorphenyl)-2(1,2,4-triazol-1-yl)-ethan.

$^1$H-NMR ($CDCl_3$): $\delta$ = 1.5-1.8 (m, 4H), 3.25 (m, 1H), 3.45 (m, 1H),
5.25 (m, 3H), 4.45 (m, 3H), 7.2-7.5 (m, 5H),
7.95 (m, 3H), 8.2 (s, 1H).

Beispiel 4 (Verb.-Nr 70)

10 g (39 mmol) 1-(2,4-Dichlorphenyl)-2-(1,2,4-triazolyl-1-yl)-ethan-1-ol werden in 30 ml 1-Chlor-4-cyclopentoxybutan gelöst. Dazu gibt man 2 g Tetrabutylammoniumhydrogensulfat und 30 ml konzentrierte Natronlauge als wäßrige Phase. Es wird über Nacht bei Raumtemperatur gerührt. Danach wird auf $H_2O$ gegossen und mehrmals mit Dichlormethan extrahiert. Die vereinigten organischen Phasen werden mit $H_2O$ neutral gewaschen und über $Na_2SO_4$ getrocknet. Nach dem Abdampfen des Lösungsmittels wird im Vakuum das noch vorhandene Alkoxyalkyhalogenid vollständig entfernt. Man erhält 11 g (73 %) 1-(4-Cyclopentoxybutoxy)-1(2,4-dichlorphenyl)-2-(1,2,4-triazol-1-yl)-ethan.

$^1$H-NMR ($CDCl_3$: = 1.4-1.8 (m, 12H), 3.2 (m, 1H), 3.35 (m, 3H),
3.85 (m, 1H), 4.2 (m, 1H), 4.45 (m, 1H),
5.05 (m, 1H), 7.2-7.4 (m, 3H), 7.9 (s, 1H),
8.15 (s, 1H).

In entsprechender Weise werden die folgenden Verbindungen hergestellt.

Tabelle 1

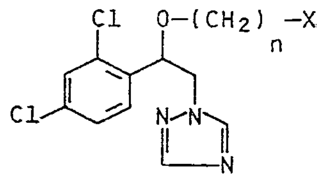

| Verb.-Nr. | X | n | IR [$cm^{-1}$] |
|---|---|---|---|
| 1 | Cl | 6 | |
| 2 | Cl | 5 | 2942, 1505, 1470, 1273 1138, 1113, 1097, 793, 679 |
| 3 | Cl | 4 | 2960, 1505, 1470, 1273, 1138, 1113, 1097, 793, 679 |
| 4 | Cl | 3 | |
| 5 | Cl | 2 | |
| 6 | F | 5 | |
| 7 | F | 4 | 2955, 1505, 1471, 1273, 1138, 1097, 1043, 793, 679 |
| 8 | F | 3 | |
| 9 | Br | 5 | |
| 10 | Br | 4 | 2950, 1505, 1470, 1273, 1138, 1097, 1043, 793 |
| 11 | Br | 3 | |
| 12 | Br | 2 | |
| 13 | CN | 5 | |
| 14 | CN | 4 | 2940, 1506, 1471, 1274, 1138, 1097, 1042, 793, 680 |
| 15 | -SCN | 4 | 2925, 2153, 1505, 1470, 1273, 1138, 1098, 1042, 793, 679 |
| 16 | $-N(C_4H_9)_2$ | 6 | |
| 17 | $-N(C_4H_9)_2$ | 5 | 2954, 2933, 2861, 1504, 1469, 1273, 1138, 1097, 1043 |
| 18 | $-N(allyl)_2$ | 5 | 2938, 1505, 1470, 1273, 1138, 1097, 1042, 920, 679 |
| 19 | $-N(allyl)_2$ | 4 | 2938, 1505, 1470, 1273, 1138, 1097, 1042, 912, 679 |
| 20 | 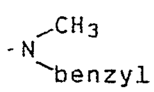 | 5 | 2940, 1504, 1469, 1452, 1273, 1138, 1097, 1043, 699 |
| 21 | $-N(C_2H_5)_2$ | 4 | |
| 22 | 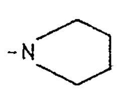 | 5 | 2935, 2874, 2788, 1505, 1470, 1273, 1138, 1097, 1043, 680 |

| Verb.-Nr. | X | n | IR [cm⁻¹] |
|---|---|---|---|
| 23 | 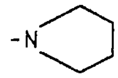 | 4 | 2949, 2874, 2788, 1505, 1470, 1273, 1138, 1096, 1042, 679 |
| 24 | 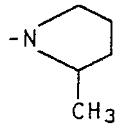 | 6 | |
| 25 | 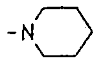 | 5 | 2935, 2857, 1505, 1470, 1273, 1138, 1097, 1042, 680 |
| 26 | 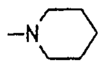 | 4 | 2935, 2854, 1505, 1470, 1273, 1138, 1097, 1042, 679 |
| 27 | 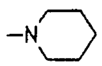 | 3 | |
| 28 | 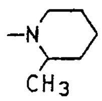 | 5 | 2933, 2859, 1505, 1470, 1273, 1138, 1097, 1043, 679 |
| 29 | 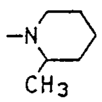 | 4 | 2933, 2862, 1505, 1470, 1273, 1138, 1097, 1043, 679 |
| 30 | 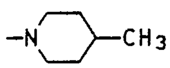 | 5 | 2946, 2925, 2867, 1505, 1496, 1273, 1137, 1097, 675 |
| 31 | 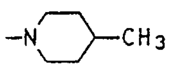 | 4 | 2947, 2923, 2869, 1505, 1469, 1273, 1137, 1097, 675 |
| 32 | 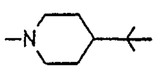 | 5 | 2941, 2865, 1504, 1469, 1364, 1273, 1137, 1097 |
| 33 | 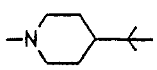 | 4 | 2944, 1868, 1505, 1470, 1364, 1273, 1138, 1097 |
| 34 | 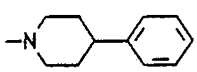 | 5 | 2936, 1505, 1470, 1273, 1138, 1097, 700, 679 |
| 35 | 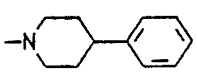 | 4 | 2937, 1505, 1470, 1273, 1138, 1097, 1042, 700 |
| 36 | 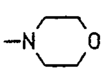 | 5 | 2941, 2859, 1505, 1470, 1273, 1139, 1118, 1097, 1042, 866 |
| 37 | 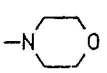 | 4 | 2948, 2858, 1505, 1469, 1273, 1138, 1118, 1097, 1042, 866 |
| 38 | 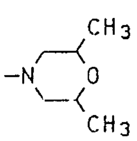 | 5 | 2935, 2865, 1505, 1463, 1273, 1142, 1097, 1080 |

| Verb.-Nr. | X | n | IR [$cm^{-1}$] |
|---|---|---|---|
| 39 | 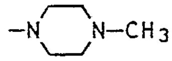 | 5 | 2937, 2795, 1505, 1469, 1273, 1165, 1139, 1096 |
| 40 | 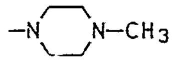 | 4 | 2935, 2794, 1505, 1460, 1273, 1166, 1139, 1097 |
| 41 | 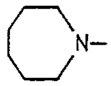 | 5 | 2929, 2858, 1504, 1470, 1273, 1137, 1096, 679 |
| 42 | 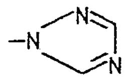 | 5 | 2944, 1506, 1470, 1273, 1139, 1097, 1042, 680 |
| 43 | 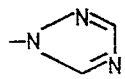 | 4 | 2950, 1506, 1470, 1273, 1140, 1097, 1042, 680 |
| 44 | 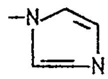 | 5 | 2940, 1506, 1273, 1138, 1097, 1042, 793, 680 |
| 45 | 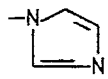 | 4 | |
| 46 | 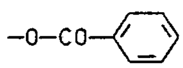 | 5 | |
| 47 | 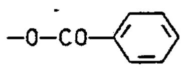 | 4 | |
| 48 | 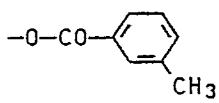 | 4 | 2950, 1716, 1505, 1279, 1201, 1087, 746 |
| 49 | 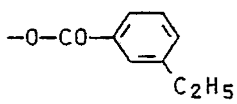 | 4 | |
| 50 | 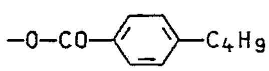 | 4 | |
| 51 | 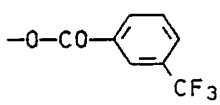 | 4 | 2950, 1723, 1337, 1257, 1169, 1130, 1096, 758, 695 |
| 52 | 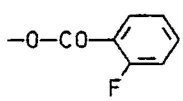 | 4 | |
| 53 | 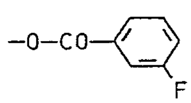 | 5 | |
| 54 | 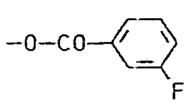 | 4 | 2950, 1720, 1591, 1447, 1281, 1204, 1096, 1069, 757 |
| 55 | 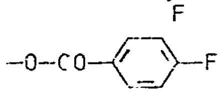 | 5 | |

| Verb.-Nr. | X | n | IR [cm⁻¹] |
|---|---|---|---|
| 56 | 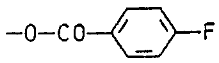 | 4 | 2950, 1718, 1604, 1507, 1274, 1153, 1112, 1093, 769 |
| 57 | 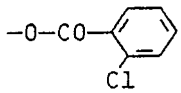 | 5 | |
| 58 | 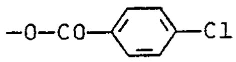 | 4 | 2950, 1718, 1593, 1273, 1094, 1015, 761 |
| 59 | 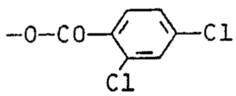 | 6 | |
| 60 | 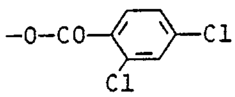 | 5 | |
| 61 | 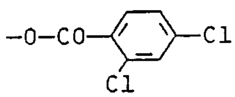 | 4 | 2950, 1730, 1586, 1470, 1376, 1286, 1244, 1137, 1099, 1048 |
| 62 | 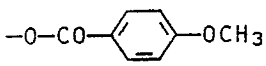 | 4 | 2955, 1709, 1607, 1511, 1275, 1258, 1168, 1099, 711 |
| 63 | 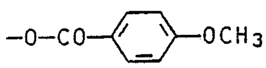 | 3 | |
| 64 | 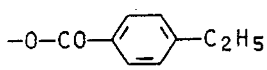 | 4 | |
| 65 | 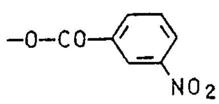 | 4 | |
| 66 | $-O-C_2H_4-C(CH_3)_3$ | 6 | 2939, 2863, 1505, 1470, 1365, 1273, 1111, 1098 |
| 67 | $-O-C_2H_4-C(CH_3)_3$ | 5 | |
| 68 | $-O-C_2H_4-CH(CH_3)-CH_2-C(CH_3)_3$ | 4 | 2953, 2865, 1469, 1365, 1273, 1116, 1098 |
| 69 | $-O-CH_2-CH(C_2H_5)-C_3H_7$ | 4 | 2956, 2928, 2867, 1504, 1468, 1273, 1197, 1110, 1097, 1048 |
| 70 | 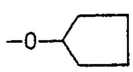 | 4 | 2953, 2869, 1505, 1471, 1273, 1138, 1096, 1042, 793, 679 |
| 71 | 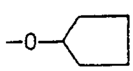 | 3 | |
| 72 | 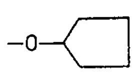 | 2 | |

| Verb.-Nr. | X | n | IR [$cm^{-1}$] |
|---|---|---|---|
| 73 | 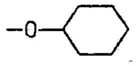 | 5 | |
| 74 | 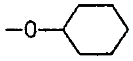 | 4 | 2932, 2855, 1448, 1273, 1108, 1096 |
| 75 | 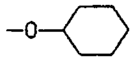 | 3 | |
| 76 | 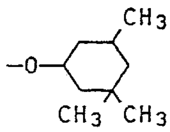 | 4 | 2951, 2923, 2867, 1460, 1365, 1105 |
| 77 | $-O-C_4H_9$ | 5 | |
| 78 | $-O-C_3H_7$ | 3 | |
| 79 | $-O-C_3H_7$ | 4 | |
| 80 | $-O-C_3H_7$ | 2 | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt-und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Alternaria solani an Kartoffeln, Tomaten,
Plasmopara viticola an Reben sowie
Fusarium-und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie

natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 10 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanol amid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III.20. Gew.-Teile der Verbindung Nr. 3 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 7 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 14 werden mit 3. Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 20 werden mit 97 Gew.-Teilen feinteiligem Kaolin innig vermischt. Man erhält auf diese Weise ein Stäubemittel, das 3 Gew.-% des Wirkstoffs enthält.

VII.30 Gew.-Teile der Verbindung Nr. 28 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII.40 Gew.-Teile der Verbindung Nr. 333 erden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 34 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebrachten werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4,-triazol,
2,3-Dicyano-1;4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorophenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl-4-ethyl-1,3-dioxolan-2-yl-ethyl]-1H-1,2,4-triazol

1-[2-(2,4-Dichlorphenyl-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Für die folgenden Versuche wurde als bekanntes Vergleichsmittel der Wirkstoff 1-[2-(2,4-Dichlorphenyl)-2-(2-propenyloxy)-ethyl]-1H-imidazol verwendet.

Anwendungsbeispiel 1

Wirksamkeit gegen Weizenmehltau

Blätter von in Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und 24 Stunden nach dem Antrocknen der Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß die Verbindungen 2, 3, 7, 10, 14 20, 28, 33, 34 und 41 bei der Anwendung als 0,025; 0,006; und 0,0015 %ige (Gew.%) Spritzbrühe eine bessere fungizide Wirkung zeigen (90 %) als das Vergleichsmittel (50 %).

Anwendungsbeispiel 2

Wirksamkeit gegen Gurkenmehltau (kurativ)

Blätter von in Töpfen gewachsenen Gurkenkeimlingen der Sorte "Chinesische Schlange" werden im Zweiblattstadium mit einer wäßrigen Konidiensuspension des Gurkenmehltaus besprüht. Nach einem Tag werden diese Pflanzen mit wäßriger Spritzbrühe, die 80 % Wirkstoff und 20 % Emulgiermittel in der Trockensubstanz enthält, bis zur Tropfnässe besprüht und im Gewächshaus bei Temperaturen zwichen 20 und 22°C und 70 bis 80 % Luftfeuchtigkeit aufgestellt. 21 Tage nach der Inoculation wird das Ausmaß des Pilzbefalles ermittelt.

Das Ergebnis des Versuches zeigt, die Verbindungen 2, 3, 7, 10, 14, 15, 19, 26, 34, 48, 51, 54, 56, 58, 61, 62, 69, 70 und 74 bei der Anwendung als 0,025 %ige Spritzbrühe eine bessere fungizide Wirkung zeigen (97 %) als das Vergleichsmittel (70 %).

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß die Wirkstoffe 2, 3 und 7 bei der Anwendung als 0,025 %ige Spritzbrühe eine bessere fungizide Wirkung zeigen (90 %) als das Vergleichsmittel (50 %).

Anwendungsbeispiel 4

Wirksamkeit gegen Pyrenophora teres

Gerstenkeimlinge der Sorte "Asse" werden im Zweiblattstadium mit wäßrigen Suspensionen, die 80 % Wirkstoff und 20 % Emulgator in der Trockensubstanz enthalten, tropfnaß gespritzt. Nach 24 Stunden werden die Pflanzen mit einer Sporensuspension des Pilzes Pyrenophora teres inoculiert und für 48 Stunden in eine Klimakammer mit hoher Luftfeuchtigkeit bei 18°C gestellt. Anschließend werden die Pflanzen im Gewächshaus bei 20 bis 22°C und 70 % relativer Luftfeuchtigkeit für weitere 5 Tage kultiviert. Dann wird das Ausmaß der Symptomentwicklung ermittelt.

Das Ergebnis des Versuches zeigt, daß die Verbindungen 2, 7, 10, 14 und 70 bei der Anwendung als 0,05 %ige Spriztbrühe eine gute fungizide Wirkung zeigen (97 %).

**Ansprüche**

1. Verbindungen der Formel I

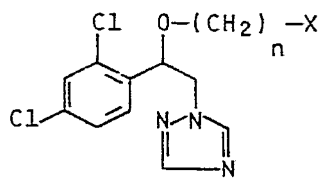

(I)

in der
n eine ganze Zahl von 2 bis 6 bedeutet und
X Halogen, Cyano, Thiocyano, gegebenenfalls $C_1$-$C_4$-alkylsubstituiertes $C_4$-$C_6$-Alkoxy, gegebenenfalls $C_1$-$C_4$-alkylsubstituiertes $C_5$-$C_8$-Cycloalkoxy, ein Amin der Formel

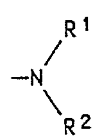

wobei $R^1$ und $R^2$ gleich oder verschieden sind und Benzyl, $C_2$-$C_4$-Alkenyl oder $C_1$-$C_4$-Alkyl bedeuten, die gegebenenfalls zu einem gegebenenfalls $C_1$-$C_4$-alkyl-oder $C_6$-$C_{10}$-arylsubstituierten Ring geschlossen sein können, der zusätzlich ein Stickstoff-oder Sauerstoffatom im Ring enthalten kann oder $-NR^1R^2$ einen Imidazolyl-oder Triazolylrest bedeutet,
oder einen Aroyloxyrest der Formel

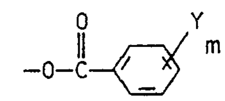

bedeutet, wobei Y für Halogen, Trifluormethyl, Nitro, $C_1$-$C_4$-Alkyl oder $C_1$-$C_4$-Alkoxy steht und m für eine ganze Zahl von 0 bis 5 steht, sowie deren für Pflanzen verträgliche Säureadditionssalze und Metallkomplexverbindungen.

2. Verbindungen der Formel I gemäß Anspruch 1, in der n 2, 3, 4, 5 oder 6 und X Fluor, Chlor, Brom, Cyano, Thiocyano, $C_4$-$C_6$-Alkoxy, ein-oder mehrfach methyl-und/oder ethylsubstituiertes $C_4$-$C_6$-Alkoxy, $C_5$-$C_6$-Cycloalkoxy, Imidazol, Triazol, Diethylamin, Di-n-butylamin, Diallylamin, Benzylmethylamin, Pyrrolidin, 2-Methylpyrrolidin, Piperidin, 2-Methylpiperidin, 4-Methylpiperidin, 4-t-Butylpiperidin, 4-Phenylpiperidin, Morpholin, 2,6-Dimethylmorpholin, Hexamethylenimin, N-Methylpiperazin, Benzoyloxy, 3-Methylbenzoyloxy, 3-Ethylbenzoyloxy, 4-Butylbenzoyloxy, 3-Trifluormethylbenzoyloxy, 4-Methoxybenzoyloxy, 4-Ethoxybenzoyloxy, 3-Fluorbenzoyloxy, 4-Fluorbenzoyloxy, 4-Chlorbenzoyloxy, 2,4-Dichlorbenzoyloxy oder 3-Nitrobenzoyloxy bedeutet.

3. Mittel zur Bekämpfung von Pilzen, enthaltend eine Verbindung gemäß Anspruch 1.

4. Mittel zur Bekämpfung von Pilzen, enthaltend eine Verbindung der Formel I

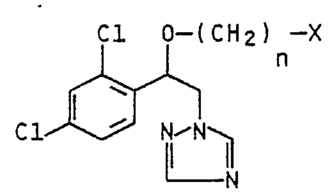

(I)

in der
n eine ganze Zahl von 2 bis 6 bedeutet und
X Halogen, Cyano, Thiocyano, gegebenenfalls $C_1$-$C_4$-alkylsubstituiertes $C_4$-$C_6$-Alkoxy, gegebenenfalls, $C_1$-$C_4$-alkylsubstituiertes $C_5$-$C_8$-Cycloalkoxy, ein Amin der Formel

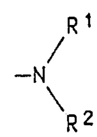

wobei $R^1$ und $R^2$ gleich oder verschieden sind und Benzyl, $C_2$-$C_4$-Alkenyl oder $C_1$-$C_4$-Alkyl bedeuten, die gegebenenfalls zu einem gegebenenfalls $C_1$-$C_4$-alkyl-oder $C_6$-$C_{10}$-arylsubstituierten Ring geschlossen sein können, der zusätzlich ein Stickstoff-oder Sauerstoffatom im Ring enthalten kann oder $-NR^1R^2$ einen Imidazolyl-oder Triazolylrest bedeutet,
oder einen Aroyloxyrest der Formel

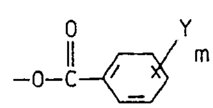

bedeutet, wobei Y für Halogen, Trifluormethyl, Nitro, $C_1$-$C_4$-Alkyl oder $C_1$-$C_4$-Alkoxy steht und m für eine ganze Zahl von 0 bis 5 steht, oder deren für Pflanzen verträgliche Säureadditionssalze oder Metallkomplexverbindungen und einen flüssigen oder festen Trägerstoff.

5. Verfahren zur Bekämpfung von Pilzen, dadurch gekennzeichnet, daß man eine Verbindung der Formel I

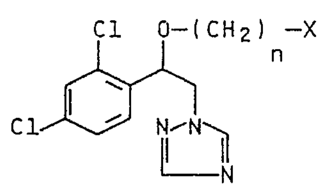

(I)

in der
n eine ganze Zahl von 2 bis 6 bedeutet und
X Halogen, Cyano, Thiocyano, gegebenenfalls $C_1$-$C_4$-alkylsubstituiertes $C_4$-$C_6$-Alkoxy, gegebenenfalls $C_1$-$C_4$-alkylsubstituiertes $C_5$-$C_8$-Cycloalkoxy, ein Amin der Formel

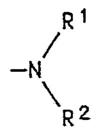

wobei $R^1$ und $R^2$ gleich oder verschieden sind und Benzyl, $C_2$-$C_4$-Alkenyl oder $C_1$-$C_4$-Alkyl bedeuten, die gegebenenfalls zu einem gegebenenfalls $C_1$-$C_4$-alkyl-oder $C_6$-$C_{10}$-arylsubstituierten Ring geschlossen sein können, der zusätzlich ein Stickstoff-oder Sauerstoffatom im Ring enthalten kann oder $-NR^1R^2$ einen Imidazolyl-oder Triazolylrest bedeutet,
oder einen Aroyloxyrest der Formel

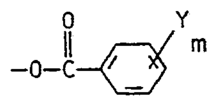

bedeutet, wobei Y für Halogen, Trifluormethyl, Nitro, $C_1$-$C_4$-Alkyl oder $C_1$-$C_4$-Alkoxy steht und m für eine ganze Zahl von 0 bis 5 steht, oder deren für Pflanzen verträgliche Säureadditionssalze oder Metallkomplexverbindungen auf diese oder auf durch Pilzbefall bedrohte Flächen, Pflanzen, Materialien oder Saatgüter einwirken läßt.

6. Verfahren zur Herstellung eines fungiziden Mittels, dadurch gekennzeichnet, daß man ein Triazolylderivat der Formel I gemäß Anspruch 1 mit festen oder flüssigen Trägerstoffen sowie gegebenenfalls mit einem oder mehreren oberflächenaktiven Mitteln mischt.

7. Verbindung der Formel I gemäß Anspruch 1, in der
n 4 oder 5 und
X Fluor, Chlor, Brom, Cyano, Hexamethylenimin oder durch $C_1$-$C_4$-Alkyl substituiertes Piperidin
bedeutet.